# EUROPÄISCHE PATENTSCHRIFT

(11) **EP 1 534 359 B1**
(45) Veröffentlichungstag und Bekanntmachung des Hinweises auf die Patenterteilung: **29.04.2009**
(21) Anmeldenummer: 03749886.2
(22) Anmeldetag: 12.05.2003
(51) Int. Cl.: A61M 1/34, A61M 1/16

(54) **VORRICHTUNG ZUR ENTFERNUNG PROTEINGEBUNDENER SUBSTANZEN**
DEVICE FOR REMOVING PROTEIN-BOUND SUBSTANCES
DISPOSITIF D'EXTRACTION DE SUBSTANCES LIEES A DES PROTEINES

(30) Priorität: 14.05.2002 EP 02010185
(43) Veröffentlichungstag der Anmeldung: 01.06.2005
(60) Teilanmeldung: 07019460.0
(73) Patentinhaber: Hepa Wash GmbH, 85748 Garching (DE)
(72) Erfinder: KREYMANN, Bernhard, Dr., 80802 München (DE)
(74) Vertreter: Graf von Stosch, Andreas
(86) Internationale Anmeldenummer: PCT/EP2003/004940
(87) Internationale Veröffentlichungsnummer: WO 2003/094998

(56) Entgegenhaltungen:
- EP-A- 0 615 780
- WO-A-84/00689
- FR-A- 2 651 438
- US-A- 4 663 049
- US-A1- 2002 019 603
- US-B1- 6 264 680

## Beschreibung

Die vorliegende Erfindung betrifft eine Vorrichtung zur Entfernung proteingebundener Substanzen aus einer biologischen Flüssigkeit, insbesondere Blut oder Blutplasma, die wenigstens ein Mittel zur Veränderung des Konzentrationsverhältnis von Toxin-Protein-Komplex gegenüber freiem Toxin und freiem Protein in der Dialysierflüssigkeit und optional der biologischen Flüssigkeit enthält. Der Begriff des Toxins wird hier sehr weit gefasst und bezieht sich im Weiteren auf alle an Proteine gebundene Substanzen, die üblicherweise nicht unmittelbar als Toxine bezeichnet werden, wie Medikamente, Elektrolyte, Hormone, Fette, Vitamine, Gase und metabolische Abbauprodukte wie Bilirubin.

Bei einer Reihe von Erkrankungen, zu denen insbesondere das akute oder chronische Nierenversagen, das akute oder chronische Leberversagen oder exogene Intoxikationen gehören, müssen pathogene Substanzen, die im Plasmawasser gelöst sind oder an Proteine gebunden sind, aus dem Blut entfernt werden. Mittels der konventionellen Verfahren der Hämodialyse, Hämofiltration oder Hämodiafiltration können proteingebundene Substanzen nur in geringem Ausmaß eliminiert werden.

Die fehlende Eliminierung proteingebundener Substanzen spielt für den Ersatz der Nierenfunktion derzeit nur eine untergeordnete Rolle. Die Lebenserwartung von Patienten mit akutem oder chronischem Nierenversagen kann durch eine Nierenersatztherapie normalisiert (bei Erholung der Organfunktion bei akutem Nierenversagen) oder zumindest für Jahre verlängert werden (bei Patienten, die eine Nierenersatztherapie weiter benötigen).

Ganz anders sieht dies bei akutem Leberversagen oder einer akuten Verschlechterung eines chronischen Leberversagens aus. Hier stehen derzeit mit der Nierenersatztherapie vergleichbare Methoden nicht zur Verfügung.

Die Leberfunktion kann im wesentlichen in zwei Hauptfunktionen unterteilt werden:
- die Synthese lebenswichtiger Proteine und
- die Entgiftung von hauptsächlich proteingebundenen Toxinen.

Für einen Ersatz der Synthesefunktion steht derzeit im Prinzip nur die Lebertransplantation zur Verfügung. Es sind zwar sogenannte Bioreaktoren mit zumindest teilweise die Synthesefunktion normaler Leberzellen übernehmenden Zellen bekannt, doch sind diese derzeit nur experimentell einsetzbar und weisen noch keine ausreichende Funktion auf. Bei ca. 20 % der Patienten mit akutem Leberversagen werden Lebertransplantationen vorgenommen, da kein adäquates Verfahren zur Übernahme der Entgiftungsfunktion existiert und damit die Zeit bis zur Erholung der Leberfunktion nicht überbrückt werden kann.

Proteingebundene Substanzen dürften in der Pathogenese der hepatischen Enzephalopathie, des hepatischen Pruritus und des hepatorenalen Syndroms eine wichtige Rolle spielen. Zu diesen pathogenen Substanzen, die vorwiegend an Albumin gebunden sind, gehören insbesondere aromatische Verbindungen wie Phenolderivate, Indolderivate, Furanderivate oder aromatische Aminosäuren, Bilirubin, C₄-C₇-Carbonsäuren, Mercaptane, Digitoxin- und Benzodiazepin-ähnliche Substanzen sowie Metallkationen wie Kupferkationen, Aluminiumkationen oder Eisenkationen. Eine der wichtigsten Erkrankungen stellt dabei die hepatische Enzephalopathie dar, da sie vital lebensbedrohlich sein kann und/oder bleibende Schäden hinterlassen kann.

Um die Entgiftungsfunktion der Leber zu ersetzen, bestehen vielfältige Ansätze seit den siebziger Jahren, die sich zum größten Teil an der Dialysetechnik orientieren:

### 1. Hämodialyse, Hämofiltration oder Hämodiafiltration

Diese konventionellen Verfahren werden derzeit bei vielen Patienten mit Leberversagen angewandt, da im letzten Stadium der Lebererkrankung in den meisten Fällen ein hepatorenales Syndrom auftritt, das auch zum dialysepflichtigen Nierenversagen führt. Durch Anwendung dieser Verfahren wird aber keine ausreichende Entgiftung proteingebundener Substanzen erzielt, sondern im wesentlichen nur die Eliminierung wasserlöslicher Substanzen mit niederem oder mittleren Molekulargewicht durchgeführt.

### 2. Hämoperfusion

Bei diesem Verfahren wird Blut oder Plasma über einen Adsorber geleitet (Kohle und/oder Kationen- oder Anionenaustauscher), um die proteingebundenen Toxine zu entfernen (O'Grady JG, Gimson AE, O'Brien CJ, Pucknell A, Hughes RD, Williams R: Controlled trials of charcoal hemoperfusion and prognostic factors in fulminant hepatic failure. Gastroenterology 94:1186-1192, 1988). Diese Methode birgt den Nachteil, dass sie unspezifisch ist und dass daher auch lebenswichtige Stoffe aus dem Blut oder Plasma entfernt werden.

### 3. Albumin als Adsorber mit zwei unterschiedlichen Verfahren

### A. MARS-System (Molecular Adsorbents Recirculating System)

Bei dem in der EP 0 615 780 beschriebenem MARS-System wird eine spezielle, mit Albumin beschichtete Dialysemembran verwendet. Die rezirkulierende albuminhaltige Dialysatflüssigkeit wird über 2 Adsorbersäulen (Kohle und Rein) geleitet, um die durch Dialyse dem Patienten entfernten eiweißgebundenen Toxine zu eliminieren, und die Bindungsstellen des Albumins im Dialysat für Toxine bereitzustellen (Stange J, Hassanein TI, Mehta R, Mitzner SR, Bartlett RH: The molecular adsorbents recycling system as a liver support system based on albumin dialysis: a summary of preclinical investigations, prospective, randomized, controlled clinical trial, and clinical experience from 19 centers. Artif Organs 26:103-110, 2002).

### B. Albumindialyse

Die Albumindialyse ist ein der kontinuierlichen Hämodialyse verwandtes Verfahren. Ein Merkmal der kontinuierlichen Nierenersatztherapie ist die Verwendung langsamer Dialysatflüsse (1-2 l/h im Vergleich zu 30 l/h bei normaler Dialyse). Im Gegensatz zur klassischen kontinuierlichen Nierenersatztherapie wird bei der Albumindialyse das Dialysat mit Albumin versetzt, so dass eine 5% Lösung entsteht (Kreymann B, Seige M, Schweigart U, Kopp KF, Classen M: Albumin dialysis: effective removal of copper in a patient with fulminant Wilson disease and successful bridging to liver transplantation: a new possibility for the elimination of protein-bound toxins. J Hepatol 31:1080-1085, 1999). Die Verwendung von Albumin beruht darauf, dass es das hauptsächliche Trägerprotein für eiweißgebundene Toxine im Blut darstellt.

Den vorstehend beschriebenen Albumin-basierenden Systemen sind für den Einsatz bei großen Patientenzahlen hohe Therapiekosten gemeinsam (Tagestherapiekosten beim MARS-System oder Albumindialyse liegen derzeit bei ca. 2500 Euro). Darüber hinaus weisen diese Systeme eine unbefriedigende Entgiftungseffizienz auf: im Durchschnitt nur eine ca. 10-30 %-ige Reduktion des Bilirubinspiegels als Marker für proteingebundene Substanzen. Zwar führen die Albumin-basierenden Dialyseverfahren zu einer Verbesserung der Symptome der hepatischen Enzephalopathie, jedoch ist eine Normalisierung der Werte auch in Folge der hohen Therapiekosten nicht erreichbar.

Es ist daher Aufgabe der vorliegenden Erfindung, eine Vorrichtung zur Dialyse bereitzustellen, mit dem in einem Schritt neben anderen unerwünschten dialysierbaren Substanzen proteingebundene Substanzen einfach und kostengünstig aus einer biologischen Flüssigkeit entfernt werden können.

Erfindungsgemäß wird diese Aufgabe durch eine Vorrichtung gemäß Patentanspruch 1 gelöst.

Die erfindungsgemäße Aufgabenlösung besteht darin, dass in einem Dialysierflüssigkeitskreislauf und optional in einem Kreislauf der biologischen Flüssigkeit mit einem geeigneten Mittel das Konzentrationsverhältnis von Toxin-Protein-Komplex gegenüber freiem Toxin und freiem Protein zugunsten des letzteren verschoben wird und sodann das Toxin entfernt wird. Analog kann es sich auch um ein Konzentrationsverhältnis von adsorbiertem Toxin gegenüber freiem Toxin und Adsorbens handeln.

Diese Lösung weist den Vorteil auf, dass die Substanzen, dadurch dass sie in Lösung gebracht werden, leicht und effizient aus der biologischen Flüssigkeit entfernt werden können.

Die US-Anmeldung Nr. 2002/0019603 A1 offenbart eine extrakorporale Vorrichtung zur Entfernung von Substanzen aus Körperflüssigkeiten. Die Vorrichtung umfasst zwei Kreisläufe, einen Kreislauf der Körperflüssigkeiten und einen zweiten Kreislauf, der chelatisierende Agenzien für die zu entfernenden Substanzen enthält und über eine Membran vom Kreislauf der Körperflüssigkeiten getrennt sein kann. An den zweiten Kreislauf (enthaltend die chelatisierenden Agenzien) kann optional ein Regenerationssystem für die Agenzien angeschlossen sein, dass bei schwach saurem pH (z.B. pH 4) eine Auflösung des Chelatkomplexes und somit Regeneration der chelatisierenden Agenzien bewirkt.

Im Rahmen dieser Erfindung werden unter gelösten Substanzen bzw. freiem Toxin sowohl Einzelmoleküle, die vom Lösungsmittel solvatisiert vorliegen, wie auch solche, die an dialysierbare Substanzen gebunden sind, verstanden. Toxine, die an dialysierbare Substanzen gebunden vorliegen, können als Komplex ebenfalls dialysierbar sein.

So ist nicht nur eine schnelle Dialyse möglich, was einen erheblichen Kostenvorteil mit sich bringt, sondern auch eine besonders umfassende Reinigung der biologischen Flüssigkeit erzielbar.

Im Fall der Reinigung von Blut oder Blutplasma führt dies darüber hinaus zu einer patientenfreundlicheren Behandlungsweise, die im Bereich der Notfallmedizin bei akut lebensbedrohenden Zuständen von entscheidender Bedeutung für den Behandlungserfolg sein kann.

Das Mittel zur Veränderung des Konzentrationsverhältnis von Toxin-Protein-Komplex gegenüber freiem Toxin und freiem Protein umfasst eine Einrichtung zur Einstellung des pH-Werts der verwendbaren Flüssigkeiten, eine Einrichtung zur Einstellung der Temperatur der verwendbaren Flüssigkeiten, eine Einrichtung zur Zugabe von Substituat zur Verdünnung oder Veränderung der Zusammensetzung (etwa des Salzgehalts) der verwendbaren Flüssigkeiten, eine Einrichtung zur Zugabe von dialysierbaren, an die zu entfernenden Substanzen bindenden Verbindungen und eine Einrichtung zur Bestrahlung der verwendbaren Flüssigkeiten mit Wellen. Im Rahmen der vorliegenden Erfindung können die unterschiedlichen Einrichtungen beliebig miteinander kombiniert werden Erfindungsgemäß wird eine Einrichtung zur Einstellung des pH-Werts der Dialysierflüssigkeit auf pH-8-13 werwendet. Es können auch wenigstens eine Einrichtung zur Einstellung des pH-Werts und wenigstens eine Einrichtung zur Einstellung der Temperatur in einem Kreislaufsystem verwendet werden.

Ein Vorteil der vorliegenden Erfindung liegt darin, dass mit einfachen Mitteln d.h. konventionellen Einrichtungen zur Zugabe von Lösungen von Säuren, Basen und optional, Substituat oder dialysierbaren Substanzen oder konventionellen Heiz-, Kühl- oder Ultraschallgeräten oder anderen Erzeugern von Licht-, Infrarot-, Ultraviolett-, oder elektromagnetischen Wellen (im Folgenden als Wellen bezeichnet), die zu entfernenden Substanzen einfach und kostengünstig in Lösung gebracht werden können, über eine Schwächung der Bindung der proteinbindenden Substanzen an Trägerproteine resp. Adsorber. Die gelösten Substanzen (Toxine) sind dialysierbar und somit einfach entfernbar.

Es wurde nämlich gefunden, dass die Bindungsaffinität von Trägerproteinen wie Albumin resp. Adsorbern zu Toxinen durch einige Maßnahmen selektiv erniedrigt werden kann, was dazu führt, dass die Konzentration der freien Toxine in der Lösung erhöht wird. Die proteingebundenen Substanzen in der biologischen Flüssigkeit oder in der Dialysierflüssigkeit stehen nämlich im Gleichgewicht zu einer geringen Menge nicht gebundener Substanzen. Durch Erniedrigung der Bindungsaffinität kann die Konzentration der nicht gebundenen, dialysierbaren Substanzen erhöht werden, wobei die freien Substanzen in Lösung gehen.

Zu diesen Maßnahmen gehören Temperaturerhöhung der Flüssigkeit, die gebundene Toxine enthält, Änderung des pH-Werts (saurer und basischer Bereich), eine Bestrahlung mit Wellen, Verdünnung der Flüssigkeit, Veränderung des Salzgehaltes und die Zuführung dialysierbarer Substanzen, die an die Toxine binden. Letzteres führt zur Bildung von dialysierbaren Komplexen.

Wenn im Dialysierflüssigkeitskreislauf wenigstens ein Mittel zur Veränderung des Konzentrationsverhältnis von Toxin-Protein-Komplex gegenüber freiem Toxin und freiem Protein vorgesehen ist, nämlich wenigstens eine Einrichtung zur Einstellung des pH-werts der Dialysierflüssigkeit auf pH = 8-13, so sollte die in der Vorrichtung verwendbare Dialysierflüssigkeit einen Adsorber für die aus der biologischen Flüssigkeit zu entfernenden Substanzen enthalten. Ein kleiner Teil der proteinbindenden Toxine in der biologischen Flüssigkeit liegt frei in Lösung vor und dieser kann durch die semipermeable Membran im Dialysator diffundieren und an die freien Bindungsstellen des Adsorbers in der Dialysierflüssigkeit binden. Anschließend wird über ein Mittel zur Veränderung des Konzentrationsverhältnis von Toxin-Protein-Komplex gegenüber freiem Toxin und freiem Protein, nämlich wenigstens eine Einrichtung zur Einstellung des pH-Werts der Dialyseflüssigkeit auf pH = 8-13, die Bindungsaffinität zwischen Adsorber und Toxin zumindest zwischenzeitlich vermindert, wodurch die zu entfernenden Substanzen in Lösung gelangen und über Dialyse (Diffusion) oder Filtration (Konvektion) oder eine Kombination beider Verfahren, im folgenden Diafiltration genannt, aus dem Dialysierkreislauf entfernt werden können.

Erfindungsgemäß ist die Trennung von Adsorber und freiem Toxin auch durch Zentrifugation möglich, wie sie z.B. in der Plasmaseparation für die Durchführung einer Plasmapherese angewendet wird. Die Zentrifugation stellt im Rahmen dieser Erfindung somit eine alternative Trennmethode zur Dialyse (als Trennmethode) dar.

Auch im Kreislauf der biologischen Flüssigkeit, die an ein Trägerprotein gebundene, zu entfernende Substanzen enthält, kann wenigstens ein erfindungsgemäßes Mittel zur Veränderung des Konzentrationsverhältnis von Toxin-Protein-Komplex gegenüber freiem Toxin und freiem Protein vorgesehen sein. So bewirkt beispielsweise die Einstellung einer erhöhten Temperatur, dass die Bindungsaffinität des Trägerproteins erniedrigt wird und die Substanzen in Lösung gehen. Dadurch können die gelösten, freien Substanzen dialysiert werden.

Sowohl der Kreislauf der biologischen Flüssigkeit als auch der Dialysierflüssigkeitskreitskreislauf kann wenigstens ein Mittel zur Veränderung des Konzentrationsverhältnis von Toxin-Protein-Komplex gegenüber freiem Toxin und freiem Protein enthalten. So enthält der Dialyseflüssigkeitskreislauf wenigstens eine Einrichtung zur Einstellung des pH-Werts der Dialyseflüssigkeit auf pH = 8-13.

Ein Vorteil der Erfindung besteht darin, dass durch die verschiedenen zur Verfügung stehenden Mittel Substanzen mit unterschiedlichem Bindungsverhalten entfernt werden können, insbesondere wenn verschiedene Maßnahmen miteinander kombiniert werden.

Ein besonders effizientes und kostengünstiges Mittel stellt die Einrichtung zur Einstellung des pH-Werts der Flüssigkeiten dar. Durch Einstellung des pH-Werts der Flüssigkeiten im basischen und ggf. auch sauren Bereich kann selektiv die Bindung unterschiedlicher Substanzen zu den Trägerproteinen resp. Adsorbern beeinflusst werden. So kann durch Zugabe einer Säure der pH-Wert der Flüssigkeit abgesenkt werden, wodurch die Bindung von gewissen Toxinen an Proteine im sauren Bereich vermindert wird und damit die Konzentration von freien Toxinen in der Flüssigkeit erhöht wird.

Beispielsweise kann derart die Bindung von Kupferionen an Albumin geschwächt werden, so dass freie, gelöste Kupferionen im darauf folgenden Filter durch Dialyse, Filtration, Diafiltration oder Zentrifugation entfernt werden können. Analog dazu kann der pH-Wert der Flüssigkeit im basischen Bereich eingestellt werden, um die im alkalischen Bereich freigesetzten Toxine anschließend durch Dialyse, Filtration oder Diafiltration aus der Flüssigkeit zu eliminieren.

Erfindungsgemäß wird durch Zugabe einer Base die Bindung von gewissen Toxinen an Proteine geschwächt und damit die Konzentration an freiem Toxin in der Dialyseflüssigkeit erhöht Erfindungsgemäß wird hierbei ein pH-Bereich von 8-13 verwendet.

Nach Entfernung der Toxine mit Mitteln der Dialyse, Filtration, Diafiltration oder Zentrifugation aus der Flüssigkeit wird der pH-Wert gegebenenfalls auf einen weiteren vorteilhaften Wert eingestellt.

Dies kann insbesondere dann einen Vorteil darstellen, wenn mit einem Adsorber, z.B. Albumin gearbeitet wird. Ein weiterer vorteilhafter pH-Wert wird dann so gewählt, dass die Affinität des Adsorbers zum Toxin wieder erhöht wird. Dies ermöglicht die Rezyklierung des Adsorbers und damit einen entscheidenden Kostenvorteil.

Ein Kreislaufsystem (für Dialysierflüssigkeit oder zu reinigende biologische Flüssigkeit) kann zwei Einrichtungen zur Einstellung des pH-Werts, ganz besonders bevorzugt drei Einrichtungen zur Einstellung des pH-Werts enthälten, um mit der ersten Einrichtung den pH-Wert im sauren resp. basischen Bereich, mit der zweiten im basischen resp. saurem Bereich und mit der dritten Einrichtung den pH-Wert wieder im ursprünglichen Bereich (in der Regel neutral) einzustellen. Weiterhin kann ein Kreislaufsystem (für Dialysierflüssigkeit oder zu reinigende biologische Flüssigkeit) zwei Einrichtungen zur Einstellung der Temperatur enthalten, so dass die verwendbare Flüssigkeit beispielsweise erwärmt und anschließend durch Kühlen wieder auf die vorherige Temperatur oder die jeweils erwünschte Temperatur gebracht werden kann. Ein Kreislaufsystem kann auch drei Einrichtungen zur Einstellung des pH-Werts und zwei Einrichtungen zur Einstellung der Temperatur enthalten.

Ein weiterer Vorteil der Erfindung besteht darin, dass mittels im Dialysierflüssigkeitskreislauf und ggf. Im Kreislauf der biologischen Flüssigkeit vorgesehener Einrichtungen zur Dialyse, Filtration oder Diafiltration die gelösten, dialysierbaren Substanzen aus den Flüssigkeiten (Dialysierflüssigkeit oder zu reinigende biologische Flüssigkeit) nach Ablösung von den Trägerproteinen resp. Adsorbern leicht und effizient entfernt werden können. Dabei können konventionelle Dialysegeräte, wie sie dem Fachmann bekannt sind, verwendet werden. Zusätzlich werden bevorzugt Vorrichtungen zur Veränderung der pH-/Temperaturwerte und Vorrichtungen zur entsprechenden Kontrolle dieser Veränderungen eingesetzt. Vorteilhafterweise wird in der erfindungsgemäßen Vorrichtung hinter eine Einrichtung zur Einstellung des pH-Werts/Temperatur der zu verwendenden Flüssigkeit eine Einrichtung zur Dialyse, Filtration, Diafiltration oder Zentrifugation geschaltet, um die freien, gelösten Substanzen direkt aus der Flüssigkeit zu entfernen. Besonders werteilhalt ist eine Vorrichtung, bei der im Dialysierflüssigkeitskreislauf und/oder im Kreislauf der biologischen Flüssigkeit in Folge eine Einrichtung zur Zugabe von Säure oder Base, eine Einrichtung zur Dialyse, Diafiltration, Filtration oder Zentrifugation, eine Einrichtung zur Zugabe von Base oder Säure, eine Einrichtung zur Dialyse, Filtration, Diafiltration oder Zentrifugation und eine Einrichtung zur Zugabe von Säure oder Base vorgesehen sind. Dadurch können unterschiedliche proteinbindende Substanzen sehr effizient aus der Dialysierflüssigkeit und der biologischen Flüssigkeit entfernt werden, und die gereinigte Dialysierflüssigkeit kann wiederum dem Dialysator zugeführt werden, um den Adsorber wiederum mit proteinbindenden Substanzen zu beladen.

Unter Einrichtungen zur Einstellung des pH-Werts fallen insbesondere Einrichtungen zur Zugabe von Säure oder Base wie beispielsweise Dosierpumpen. Als Säuren oder Basen bieten sich wässrige Lösungen von biologisch verträglichen Säuren oder Basen an. Allgemein bevorzugt werden Säuren oder Basen eingesetzt, deren konjugierte Basen oder Säuren im menschlichen Organismus natürlich auftretende Ionen darstellen. Beispielsweise können als Säuren Salzsäure, Schwefelsäure oder Essigsäure verwendet werden, bevorzugt Salzsäure. Als Basen können beispielsweise Natronlauge oder Kalilauge eingesetzt werden, bevorzugt Natronlauge. Die Dialyseflüssigkeit wird durch Zugabe von Base auf einen pH = 8-13 eingestellt. Die biologische oder Dialyse-Flüssigkeit kann beispielsweise auch durch Zugabe von Säure auf einen pH-Wert zwischen 1 und 7, vorteilhaft zwischen 2.5 und 5, und durch Zugabe von Base auf einen pH-Wert zwischen 7 und 13 oder zwischen 8 und 13 eingestellt werden. Der jeweils gewünschte pH-Wert richtet sich im wesentlichen nach der Beschaffenheit der verwendeten Flüssigkeit, der Beschaffenheit des Proteins und den Eigenschaften der zu entfernenden Substanzen. Beispielsweise ist die Bindungsaffinität von Kupfer an Albumin im pH-Bereich von etwa zwei signifikant erniedrigt. Umgekehrt betrachtet bedeutet dies, dass bei einem pH-Wert von größer als etwa 3 eine besonders große Bindungsaffinität von Kupfer an Albumin vorliegt.

Außerdem wurde beispielsweise beobachtet, dass die Bindungsaffinität von Bilirubin an Albumin bei einem pH-Wert von etwa 12 signifikant erniedrigt ist.

Unter Einrichtung zur Einstellung der Temperatur fallen insbesondere Einrichtungen zum Erwärmen wie übliche Heizgeräte, Mikrowellengeräte oder auch Infrarotgeräte oder konventionelle Kühlaggregate als Einrichtung zum Kühlen. Im Dialysierflüssigkeitskreislauf und/oder der Kreislauf der biologischen Flüssigkeit können jeweils eine oder mehrere Einrichtungen zum Erwärmen/Kühlen angeordnet sein. Insbesondere können durch Erwärmung oder Kühlen der verwendbaren Flüssigkeiten die zu entfernenden Substanzen in Lösung gebracht werden, während mittels Kühlen oder Erwärmen die biologische Flüssigkeit oder die Dialysierflüssigkeit wieder auf die gewünschte Temperatur gebracht werden können. Die Art und der Umfang des verwendeten Temperaturgradienten hängt von der Art der Flüssigkeit, vom Adsorber sowie vom zu entfernenden Toxin ab. Beispielsweise ist es möglich, zunächst aufzuheizen und anschließend wieder zu kühlen. Auch der umgekehrte Vorgang kann vorteilhaft sein. Ebenso kann es vorteilhaft sein, die Aufheizung/Abkühlung stufenweise vorzunehmen.

Ein weiterer Vorteil der Erfindung besteht darin, dass durch eine Einrichtung zum Kühlen Erwärmen der verwendbaren Dialysierflüssigkeit die Bindungsaffinität des Adsorbers selektiv erhöht werden kann, wodurch freie, gelöste Substanzen, die in die Dialyseflüssigkeit diffundiert sind, durch rezyklierte Adsorber gebunden werden können.

Die gewünschte Temperatur der zu verwendenden Flüssigkeiten hängt im wesentlichen von deren Beschaffenheit ab. Wird als biologische Flüssigkeit Blut oder Teilprodukte des Bluts wie Blutplasma oder Fraktionen davon eingesetzt, so kann auf eine Temperatur bis auf ca. 150 °C (mit einer entsprechenden Druckerhöhung gekoppelt, wie sie z.B. bei der Hitzesterilisation von Milch verwendet wird) bevorzugt bis 45 °C erwärmt werden. Eine Erwärmung über den physiologischen Bereich hinaus ist also ebenfalls möglich. Bei Verwendung der erfindungsgemäßen Dialysevorrichtung in einem extrakorporalen Kreislauf an einem Patienten kann die Temperatur wieder auf einen für den Patienten optimalen Temperaturwert im Bereich von 35 bis 37 °C, bei Patienten mit hepatischer Enzephalopathie ca. 35 °C elsius, herabgesenkt werden.

Wird die Einrichtung zur Einstellung der Temperatur im Kreislauf der Dialysierflüssigkeit angewendet, kann die Temperatur je nach Zusatz, z.B. von Dampf oder einer Druckerhöhung oder anderen Stabilisatoren (bekannt aus der Behandlung von Albumin zur Pasteurisierung) auch auf über 150 °C erhöht werden.

Die Erwärmung der zu verwendenden Flüssigkeit im Kreislaufsystem kann über eine direkte Erwärmung des flüssigkeitsgefüllten Schlauchsystems mittels eines Heizgeräts oder mittels Bestrahlung mit Mikrowellen oder Infrarot durchgeführt werden. Aufgrund des Wärmeaustauschs zwischen der zu reinigenden Flüssigkeit und der Dialysierflüssigkeit im Dialysator kann es beispielweise ausreichend sein, nur im Dialysierflüssigkeitskreislauf Einrichtungen zum Erwärmen zu enthalten, wobei trotzdem eine Erwärmung der biologischen Flüssigkeit auftritt. Gemäß einer weiteren Ausführungsform kann eine Einrichtung zum Erwärmen auch vor dem Eintritt in den Dialysierflüssigkeitskreislauf oder den Kreislauf der biologischen Flüssigkeit einer Einrichtung zur Einstellung des pH-Werts oder einer Einrichtung zur Zugabe von Substituat nachgeschaltet sein. In diesem Fall werden die Dialysierflüssigkeit und/oder die zu reinigende Flüssigkeit durch Zugabe von angewärmten Lösungen erwärmt.

Als Einrichtungen zur Bestrahlung mit Wellen kann beispielsweise ein Ultraschallgerät verwendet werden. Weitere geeignete Einrichtungen sind solche, die zur Erzeugung von Licht-, Ultraviolett, Infrarot, Radio- und Mikrowellen geeignet sind.

Als Mittel zur Veränderung des Konzentrationsverhältnis von Toxin-Protein-Komplex gegenüber freiem Toxin und freiem Protein bietet sich auch eine Einrichtung zur Zugabe von dialysierbaren, an die zu entfernenden Substanzen bindenden Verbindungen. Dabei kann es sich um konventionelle Dosierpumpen handeln, die wässrige Lösungen der dialysierbaren Verbindungen zuführen. Die dialysierbaren Substanzen, die zum Teil toxingebunden vorliegen, können über die konventionellen Einrichtungen zur Dialyse oder Diafiltration einfach entfernt werden.

Als bindende Verbindungen können dialysierbare Verbindungen mit niedrigem/mittlerem Molekulargewicht, die sich durch eine starke Affinität zu den zu entfernenden Substanzen auszeichnen, verwendet werden. Zu den bevorzugten Verbindungen gehören Coffein, das an Bilirubin bindet, und gängige Chelatbildner wie Penicillamin, Trientine, Deferoxamin, Preferiprone, HBED, Vitamin C, BAL, DMPS oder DMSA, die an Metallkationen wie beispielsweise Kupferionen oder Eisenionen binden. Die dialysierbaren Verbindungen können sowohl der biologischen Flüssigkeit als auch der Dialysierflüssigkeit zugefügt werden, bevorzugt erfolgt die Zugabe aber in die Dialysierflüssigkeit, um Komplikationen durch mögliche Verunreinigungen der biologischen Flüssigkeit bei unvollständiger Entfernung durch Dialyse zu vermeiden.

Darüber hinaus können bei der Verwendung zweier Mittel zur Veränderung des Konzentrationsverhältnisses von Toxin-Protein-Komplex gegenüber freiem Toxin und freiem Protein, wie zum Beispiel pH-Wert-Erhöhung und Zugabe einer bindenden Verbindung (z.B. Coffein) auch synergistische Effekte entstehen.

Zu einer Einrichtung zur Zugabe von Substituat zur Verdünnung oder Veränderung des Salzgehalts der verwendbaren Flüssigkeiten gehören übliche Dosierpumpen, mit denen eine Substituatlösung zugegeben werden kann. Bevorzugt wird solch eine Einrichtung in Kombination mit einer Einrichtung zum Erwärmen, die dieser nachgeschaltet ist, verwendet, so dass in den Kreislauf der verwendeten Flüssigkeiten angewärmtes Substituat zugegeben wird. Als Substituatlösung kommen wässrige Lösungen, die verschiedene Salze sowie auch Harnstoff enthalten können, in Frage. Dabei kann es sich um kommerzielle Dialysierflüssigkeiten handeln, die je nach Bedarf durch Zufügen von Salzen auf die erwünschte Konzentration eingestellt werden können. In Frage kommen aber auch, wie oben erwähnt, Stabilisatoren, Mittel zur Blutverdünnung wie Heparin oder Citrat oder Substanzen zur Veränderung des osmotischen Gleichgewichts, wie Salze, oder des elektrophysiologischen Gleichgewichts (Donnan-Effekt) wie negativ oder positiv geladene Substanzen. Das Substituat dient nicht nur dazu, die zu entfernenden Substanzen durch Veränderung der Salzkonzentration in der Flüssigkeit in Lösung zu bringen. Die Salzkonzentration der biologischen Flüssigkeit, z.B. Blut, kann durch Substituatzugabe auch genau dem Zustand des Patienten entsprechend eingestellt werden. Zudem kann dadurch im Dialysierkreislauf die Bindungsfähigkeit von rezykliertem Adsorber für Toxine wiederhergestellt werden. Zusätzlich kann die Zugabe von Harnstoff für eine bessere Bindungsfähigkeit des Adsorbers notwendig sein.

Als Dialysator können konventionelle, zur Zeit z.B. für die Hämodialyse verwendete Dialysatoren eingesetzt werden. Es ist aber auch vorstellbar, dass Membranen mit größeren Poren als derzeit für die Dialyse verwendet werden, in Frage kommen. Der Dialysator ist mit einer konventionellen, semipermeablen Dialysemembran ausgestattet, gegebenenfalls kann die Diffusion durch die Membran durch konvektiven Transport mittels Filtration unterstützt werden. Im wesentlichen umfasst der Dialysator zwei Kammern, die durch eine Dialysemembran getrennt sind, an die jeweils ein Kreislaufsystem (Schlauchsystem) für die zu verwendenden Flüssigkeiten angeschlossen ist. Die zu reinigende biologische Flüssigkeit und die Dialysierflüssigkeit werden üblicherweise im Gegenstromverfahren geführt. Möglich ist aber auch die Durchführung eines Gleichstromverfahrens. Übliche Ausgestaltungen eines Dialysegeräts wie Manometer, Luftdetektoren, Pumpeneinrichtungen wie Heparinpumpen, Blutpumpen usw. werden von der erfindungsgemäßen Vorrichtung mitumfasst. Mit der erfindungsgemäßen Vorrichtung können sowohl langsame (1-2 l/h) als auch normale Dialysatflüsse (25-50 l/h) erzielt werden, wie auch Geschwindigkeiten dazwischen je nach Bedarf.

Zu den biologischen Flüssigkeiten, die in der erfindungsgemäßen Vorrichtung resp. im erfindungsgemäßen Verfahren verwendet werden können, gehören alle Körperflüssigkeiten von Menschen oder Tieren, insbesondere Blut oder Blutplasma, besonders bevorzugt humaner Abstammung. Neben der Entfernung von proteingebundenen Substanzen aus den verwendeten biologischen Flüssigkeiten erfolgt gleichzeitig eine Eliminierung üblicherweise bei der konventionellen Dialyse entfernbarer, wasserlöslicher Substanzen wie beispielsweise Harnstoff oder verschiedener Ionen. Die zu entfernenden proteinbindenden Substanzen sind bevorzugt an das Trägerprotein Albumin gebunden. Die erfindungsgemäße Vorrichtung eignet sich insbesondere zur Blut- und Plasmareinigung im Bereich der Medizin, und kann sowohl im Bereich der Blutkonservenverarbeitung wie auch bei extrakorporaler Dialyse an Patienten eingesetzt werden.

Als Dialysierflüssigkeiten können konventionelle Dialysierflüssigkeiten, wie sie dem Fachmann bekannt sind, verwendet werden. Die Ionenkonzentration kann jeweils an den Bedarf des Patienten angepasst werden. Je nach Bedarf können gebräuchliche ionenhaltige wässrige Lösungen oder reines Wasser verwendet werden. Gegebenenfalls werden die konventionellen Dialysierflüssigkeiten mit einem Adsorber für die zu entfernenden proteinbindenden Substanzen versehen. Als Adsorber kommen beispielsweise Resinat und Akzeptorproteine in Frage. Ein bevorzugtes Akzeptorprotein ist Albumin, dieses kann humanes Serumalbumin, tierisches Albumin oder auch gentechnisch hergestelltes Albumin sein. Besonders bevorzugt wird humanes Serumalbumin eingesetzt. Die Serumalbumin-Lösungen können gegebenenfalls mit Wasser oder konventionellen Dialysierflüssigkeiten, oder anderen Flüssigkeiten verdünnt werden. Die verwendete Dialysierflüssigkeit kann bevorzug humanes Serumalbumin in einer Konzentration von 0.1 bis 25 g pro 100 ml, bevorzugt 2 bis 10 g pro 100 ml, besonders bevorzugt 4 bis 6 g pro 100 ml, enthalten.

Des weiteren können als Dialysierflüssigkeit auch Blut, Blutserum oder first getrorenes Plasma (fresh frozen plasma) verwendet werden. Bei der Dialysierflüssigkeit kann es sich auch um Dialysat aus einem Bioreaktor handeln. Derzeit sind für Bioreaktoren (mit lebenden Leberzellen arbeitende Systeme zur Leberersatztherapie) enorme Blutmengen notwendig; so muss dem Patienten während der Zirkulation des Bioreaktors bis zu einem Liter Blut aus dem Kreislauf entnommen werden. Zur Anregung der Synthesefunktion der Leberzellen des Bioreaktors kann aber auch ein System ausreichend sein, bei dem ein Dialysat, das Substanzen enthält, die normalerweise in der Leber entgiftet werden, zur Anregung eingesetzt wird. Entsprechend kann ein wie unter 1 und 2 beschriebenes Dialysat zunächst im extrakorporalen Kreislauf über den Bioreaktor geleitet werden. Dieses Dialysat wird dann wie unter 2 beschrieben gereinigt und das Dialysat dem Patienten wieder zugeführt. Hierzu kann es notwendig sein, dem Dialysat kontinuierlich Albumin zuzuführen oder aber eine Kapillare resp. eine Membran zu benutzen, die für Albumin durchlässiger ist als derzeit verwendete Dialysefilter.

Die erfindungsgemäße Vorrichtung kann mit einem oder mehreren konventionellen pH-Messgeräten und/oder Temperaturmeßgeräten ausgestattet sein zur Überwachung der entsprechenden Eigenschaften der verwendeten Flüssigkeiten.

Beschrieben wird weiterhin ein Verfahren zur Entfernung von unerwünschten Substanzen aus einer biologischen Flüssigkeit zur Ex-vivo-Reinigung biologischer Flüssigkeit umfassend das Dialysieren einer biologischen Flüssigkeit gegen eine Dialysierflüssigkeit durch eine semipermeable Membran, wobei die Dialysierflüssigkeit einen Adsorber für zu entfernende proteinbindende Substanzen enthält, und die Dialysierflüssigkeit und optional

die biologische Flüssigkeit durch Zugabe von Säure, Base oder dialysierbarer Substanzen, durch Verdünnung, Veränderung des Salzgehalts, Bestrahlung mit Wellen oder Erwärmung so eingestellt wird, dass die Bindungsaffinität des Trägerproteins zu den gebundenen, zu entfernenden Substanzen erniedrigt wird und dadurch die Konzentration der freien unerwünschten Substanzen erhöht wird. Dabei wird der plt-Wert des verwendbaren dialysierflüssigkeit intermediar auf Ph=8-13 eingestellt.

Vorteilhaft ist außerdem ein Verfahren, bei dem ein Kreislaufsystem verwendet wird, wobei das Verfahren eine mindestens zweimalige Zugabe von Säure, Base oder dialysierbarer Substanzen, Verdünnung, Veränderung des Salzgehalts, Bestrahlung mit Wellen oder Erwärmung/Kühlen der Dialysierflüssigkeit oder der biologischen Flüssigkeit verwendet.

Die beschriebenen Verfahren und Vorrichtungen können allgemein zur Reinigung biologischer Flüssigkeiten verwendet werden. Zu biologischen Flüssigkeiten gehören alle Körperflüssigkeiten von Menschen oder Tieren, insbesondere Blut oder Blutplasma, besonders bevorzugt humaner Abstammung. Dabei ist es möglich, die entnommenen Flüssigkeiten, insbesondere Blut oder Blutplasma, dem Körper wieder zurückzuführen, oder für andere Zwecke zugänglich zu machen. So können beispielsweise Blutkonserven gereinigt werden, oder die gereinigten biologischen Flüssigkeiten werden weiteren kommerziellen Zwecken oder Forschungszwecken zugeführt.

Zur Durchführung der Verfahren ist die vorstehend beschriebene Vorrichtung geeignet. Weitere Einzelheiten, Merkmale und Vorteile des Verfahrens ergeben sich aus der vorstehenden Besprechung der Vorrichtung und den Ansprüchen.

Nachfolgend werden unter Bezugnahme auf die Figuren drei Ausführungsbeispiele der Erfindung näher erläutert. Die Figuren zeigen besondere Ausführungsformen der erfindungsgemäßen Vorrichtung in schematischer Darstellung.

Es zeigen:
Figur 1 eine vereinfachte schematische Darstellung einer Ausführungsform der erfindungsgemäßen Vorrichtung mit Einrichtungen zum Erwärmen und Kühlen und einer Einrichtung zur Substituatzugabe im extrakorporalem Kreislauf, wobei lediglich der Kreislauf der biologischen Flüssigkeit gezeigt ist.
Figur 2 eine vereinfachte schematische Darstellung einer Ausführungsform der erfindungsgemäßen Vorrichtung mit Einrichtungen zur Einstellung des pH-Werts im extrakorporalem Kreislauf, wobei lediglich der Kreislauf der biologichen Flüssigkeit gezeigt ist.
Figur 3 eine vereinfachte schematische Darstellung einer Ausführungsform der erfindungsgemäßen Vorrichtung mit Einrichtungen zum Erwärmen und Kühlen, Einrichtungen zur Einstellung des pH-Werts und einer Einrichtung zur Substituatzugabe im Dialysierflüssigkeitskreislauf.

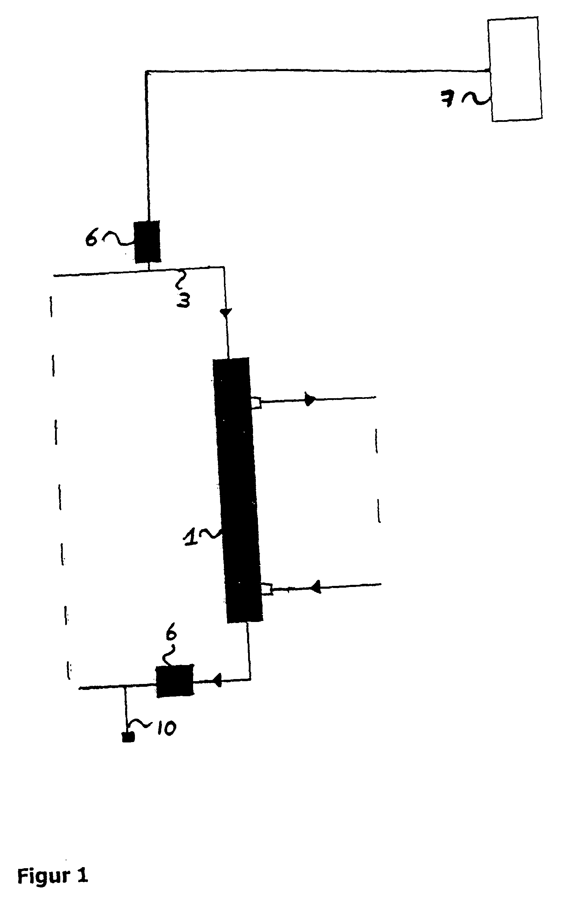

Figur 1 zeigt eine Vorrichtung zur Hämodialyse bestehend im wesentlichen aus einem Dialysator (1), einem Dialysierflüssigkeitskreislauf (2) (in der Figur nur angedeutet, einem Blutkreislauf (3) (in der Figur nur andeutungsweise eingezeichnet), Heiz- und Kühlgeräten (6), einer Einrichtung zur Substituatzugabe (7) und einem Temperaturmessgerät (10).

Das Blut wird im Blutkreislauf (3) vor Eintritt in den Dialysator (1) über die Einrichtung (7) mit im Heizgerät (6) angewärmten Substituat aus z.B. konventioneller Hämofiltrationslösung versetzt. Anschliessend gelangt das angewärmte Blut in die Blutkammer des Dialysators (1). Aufgrund der erhöhten Temperatur des Bluts kommt es zu einer erhöhten Freisetzung von proteingebundenen Substanzen von den Trägerproteinen, und somit liegt ein erhöhter Pool gelöster, dialysierbarer Toxine vor, die über die Dialysemembran in die Dialysekammer des Dialysators (1) diffundieren. Nachdem das von proteingebundenen Substanzen gereinigte Blut den Dialysator (1) verlassen hat, wird es durch das Kühlaggregat (6) wieder abgekühlt auf eine physiologisch annehmbare Temperatur, die mittels dem Temperaturmessgerät (10) überprüft wird. Alternativ kann die Bluttemperatur und damit die Temperatur des Patienten auch durch die Steuerung der Dialysattemperatur eingestast werden Anschließend wird das Blut im Blutkreislauf (3) zurückgeführt.

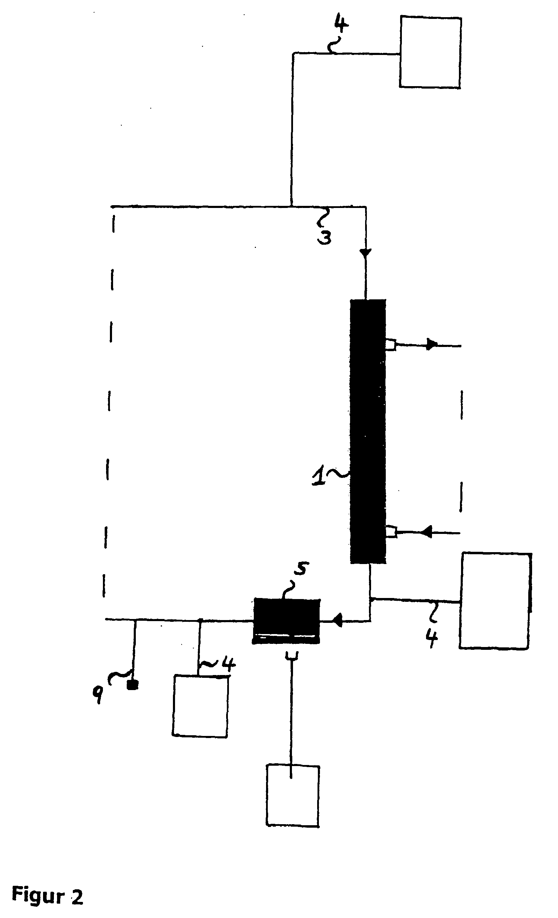

Figur 2 zeigt eine Vorrichtung zur Hämodialyse bestehend im wesentlichen aus einem Dialysator (1), einem Dialysierflüssigkeitskreislauf (2) (in der Figur nur angedeutet einem Blutkreislauf (3) (in der Figur nur andeutungsweise eingezeichnet), Dosierpumpen zur Säure- oder Basenzugabe (4), Dialysator (5) und ein pH-Messgerät (9). Das Blut wird im Blutkreislauf (3) vor Eintritt in den Dialysator (1) mittels der Dosierpumpe (4) mit HCl-Lösung versetzt. Dadurch wird der pH-Wert des Bluts abgesenkt, und ein Teil der Toxine geht in Lösung. Anschließend gelangt das angesäuerte Blut in die Blutkammer des Dialysators (1). Die gelösten dialysierbaren Substanzen können über die Dialysemembran in die Dialysekammer des Dialysators (1) diffundieren. Nachdem das teilweise von proteingebundenen Substanzen befreite Blut den Dialysator (1) verlassen hat, wird es mittels der Dosierpumpe (4) mit NaOH-Lösung versetzt, wodurch der pH-Wert im basischen Bereich eingestellt wird und weitere proteinbindende Toxine in Lösung gehen. Stromabwärts gelangt das Blut in einen weiteren Dialysator (5). Dort wird erneut entweder eine Dialyse, Filtration oder eine Diafiltration durchgeführt, um im alkalischen Bereich gelöste, sonst proteingebundene Substanzen zu eliminieren. Anschließend wird der pH-Wert über eine Dosierpumpe (4) mit HCl-Lösung im neutralen Bereich bei ca. 7.4 eingestellt, was mittels dem pH-Messgerät (2) überprüft wird. Daraufhin wird das Blut im Blutkreislauf (3) zurückgeführt.

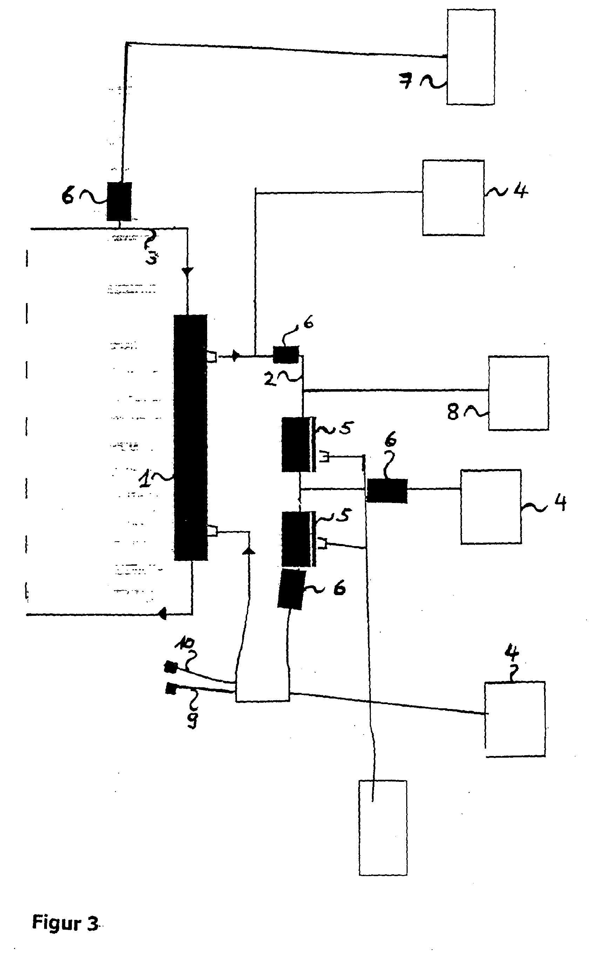

Figur 3 zeigt eine Vorrichtung zur Hämodialyse bestehend im wesentlichen aus einem Dialysator (1), einem Dialysierflüssigkeitskreislauf (2), einem Blutkreislauf (3) (in der Figur nur andeutungsweise eingezeichnet), Dosierpumpen zur Säure- oder Basenzugabe (4), Dialysatoren (5), Heiz- und Kühlgeräten (6), einer Einrichtung zur Substituatzugabe (7), einer Einrichtung zur Coffeinzugabe (8), einem pH-Messgerät (9) und einem Temperaturmessgerät (10).

Das Blut wird im Blutkreislauf (3) vor Eintritt in den Dialysator (1) über die Einrichtung (7) mit im Heizgerät (6) angewärmten Substituat aus z.B. Hämofiltrationslösung versetzt. Anschliessend gelangt das angewärmte Blut in die Blutkammer des Dialysators (1). Aufgrund der erhöhten Temperatur des Bluts liegt ein erhöhter Pool freier, dialysierbarer Toxine vor, die über die Dialysemembran in die Dialysekammer des Dialysators (1) diffundieren. Zudem enthält die Dialysierflüssigkeit Albumin, das an die Toxine bindet, so dass der Pool freier Substanzen in der Dialysierflüssigkeit niedrig gehalten wird, wodurch die Diffusion der Toxine in die Dialysierflüssigkeit verstärkt wird. Nachdem das von proteingebundenen Substanzen gereinigte Blut den Dialysator (1) verlassen hat, wird es im Blutkreislauf (3) zurückgeführt.

Die Dialysierflüssigkeit aus dem Dialysator (1), die albumingebundene Toxine enthält, gelangt in den Dialysierflüssigkeitskreislauf (2). Über die Dosierpumpe (4) wird der Dialysierflüssigkeit HCl-Lösung zugefügt. Dadurch wird der pH-Wert der Dialysierflüssigkeit herabgesenkt, und der Pool gelöster, freier Toxine in der Flüssigkeit steigt an. Stromabwärts ist im Dialysierflüssigkeitskreislauf (2) ein Heizgerät (6) angeordnet, das die Dialysierflüssigkeit auf 41-45°C erwärmt, wodurch der Pool freier Toxine weiter erhöht wird und der Anteil proteingebundener Toxine absinkt. Anschließend ist im Kreislauf (2) eine Dosierpumpe (8) für Coffein angebracht. Durch die Zugabe von Coffein wird insbesondere Bilirubin gebunden, und dadurch erniedrigt sich der Anteil des proteingebundenen Bilirubins in der Dialysierflüssigkeit. Stromabwärts gelangt die Dialysierflüssigkeit in einen Dialysator (5). Dort wird dem System ein Teil der Dialysierflüssigkeit entzogen, um die Konzentration des Adsorbers im gewünschten Bereich zu halten. Zusätzlich wird durch Dialyse, Filtration oder Diafiltration eine Reinigung des Dialysates durchgeführt, insbesondere von freien, proteinbindenden substanzen und coffein-gebunden Bilirubin. Das Albumin kann aufgrund seiner hohen Molmasse den Filter nicht passieren. Nach dem Austritt aus dem Dialysator (5) ist im Dialysierflüssigkeitskreislauf (2) eine Dosierpumpe (4) zur Zugabe von NaOH-Lösung angeordnet, wobei vor Eintritt in den Kreislauf ein Heizgerät (6) angeordnet ist. Stromabwärts folgt wiederum ein Dialysator (5), der dem System die zugeführte Flüssigkeit entzieht und im alkalischen Bereich gelöste Substanzen durch Dialyse, Filtration oder Diafiltration eliminiert. Anschliessend ist im Kreislauf (2) eine Kühlvorrichtung (6) angebracht, mit der die Temperatur der Dialysierflüssigkeit entsprechend der gewünschten Patiententemperatur angepasst werden kann. Über die nachfolgende Dosierpumpe (4) wird der Dialysierflüssigkeit HCl-Lösung zugefügt, um den pH-Wert der Dialysierflüssigkeit im Neutralen einzustellen, so dass die Bindungsfähigkeit von Albumin wieder erhöht wird und keine negative Beeinflussung des pH-Werts des Blutes im Dialysator auftritt. Anschliessend sind im Kreislauf (2) ein pH-Messgerät (9) und ein Temperaturmessgerät (10) angeordnet, um den pH-Wert und Temperaturwert der gereinigten Dialyseflüssigkeit vor Wiedereintritt in den Dialysator (1) zu überprüfen.

## Patentansprüche

1. Vorrichtung zur Dialyse einer biologischen Flüssigkeit, die zu entfernende proteinbindende Substanzen enthält, mit
- einem Kreislauf (3) der biologischen Flüssigkeit und
- einem Dialysator (1), wobei im Dialysierflüssigkeitskreislauf (2)
- wenigstens ein Mittel (4;6;7;8;9) zum Inlösungbringen der zu entfernenden proteinbindenden Substanzen und
- wenigstens eine Einrichtung (5) zur Dialyse, Filtration oder Diafiltration vorgesehen ist,
wobei eine in der Vorrichtung verwendbare Dialysierflüssigkeit einen Adsorber
für die aus der biologischen Flüssigkeit zu entfernenden Substanzen enthält, **dadurch gekennzeichnet, dass** das Mittel (4;6;7;8;9) zum Inlösungbringen der zu entfernenden proteinbindenden Substanzen mindestens eine Einrichtung (4) zur Einstellung des pH-Werts der verwendbaren Dialysierflüssigkeit auf pH = 8 - 13 umfasst.

2. Vorrichtung gemäß Anspruch 1 umfassend mindestens ein weiteres Mittel (4;6;7;8;9) zum Inlösungbringen von zu entfernenden proteinbindenden Substanzen ausgewählt aus der Gruppe
- Einrichtung (4) zur Einstellung des pH-Werts der verwendbaren Flüssigkeiten auf pH = 2,5 - 5,
- Einrichtung (6) zur Einstellung der Temperatur der verwendbaren Flüssigkeiten,
- Einrichtung (7) zur Zugabe von Substituat zur Verdünnung oder Veränderung des Salzgehalts der verwendbaren Flüssigkeiten,
- Einrichtung (8) zur Zugabe von dialysierbaren, an die zu entfernenden Substanzen bindenden Verbindungen,
- Einrichtung (9) zur Bestrahlung der verwendbaren Flüssigkeiten mit Wellen.

3. Vorrichtung gemäß Anspruch 1 oder 2, **dadurch gekennzeichnet, dass** in dem Kreislauf (3) der biologischen Flüssigkeit wenigstens ein Mittel (4;6;7;8;9) zum Inlösungbringen von zu entfernenden proteinbindenden Substanzen vorgesehen ist.

4. Vorrichtung gemäß einem der voranstehenden Ansprüche, **dadurch gekennzeichnet, dass** die Einrichtung (4) zur Einstellung des pH-Werts der verwendbaren Flüssigkeiten eine Einrichtung zur Zugabe von Base und eine Einrichtung zur Zugabe von Säure umfasst.

5. Vorrichtung gemäß einem der voranstehenden Ansprüche, **dadurch gekennzeichnet, dass** die Einrichtung (6) zur Einstellung der Temperatur Einrichtungen zum Erwärmen oder Kühlen umfasst.

6. Vorrichtung gemäß Anspruch 5, **dadurch gekennzeichnet, dass** die Einrichtung (6) zum Erwärmen ein Heizgerät, ein Mikrowellengerät oder ein Infrarotgerät umfasst und die Einrichtung (6) zum Kühlen ein Kühlaggregat darstellt.

7. Vorrichtung gemäß Anspruch 2, **dadurch gekennzeichnet, dass** die Einrichtung (9) zum Bestrahlen ein Ultraschallgerät umfasst.

8. Vorrichtung gemäß einem der voranstehenden Ansprüche, **dadurch gekennzeichnet, dass** im Kreislauf (3) der biologischen Flüssigkeit wenigstens eine weitere Einrichtung (5) zur Dialyse, Filtration oder Diafiltration vorgesehen ist.

9. Vorrichtung gemäß einem der voranstehenden Ansprüche, **dadurch gekennzeichnet, dass** im Kreislauf (3) der biologischen Flüssigkeit eine Einrichtung (6) zum Erwärmen der biologischen Flüssigkeit bis zu 45°C und eine Einrichtung (6) zum Kühlen der verwendbaren Flüssigkeiten vorgesehen sind.

10. Vorrichtung gemäß einem der vorhergehenden Ansprüche, **dadurch gekennzeichnet, dass** die Einrichtung (6) zum Erwärmen vor dem Eintritt in den Dialysierflüssigkeitskreislauf (2) oder den Kreislauf (3) der biologischen Flüssigkeit einer Einrichtung (4) zum Einstellen des pH-Werts oder einer Einrichtung (7) zur Zugabe von Substituat nachgeschaltet ist.

11. Vorrichtung gemäß einem der vorhergehenden Ansprüche, **dadurch gekennzeichnet, dass** der Adsorber Albumin umfasst.

12. Vorrichtung gemäß Anspruch 11, **dadurch gekennzeichnet, dass** die Dialysierflüssigkeit humanes Serumalbumin in einer Konzentration von 1 bis 25 g pro 100 ml, bevorzugt 2 bis 10 g pro 100 ml, besonders bevorzugt 4 bis 6 g pro 100 ml, enthält.

13. Vorrichtung zur Dialyse einer biologischen Flüssigkeit, die zu entfernende proteinbindende Substanzen enthält, gemäß einem der Ansprüche 1 - 10, wobei die in der Vorrichtung verwendbare Dialysierflüssigkeit keinen Adsorber für die aus der biologischen Flüssigkeit zu entfernenden Substanzen enthält.

## Claims

1. Means for the dialysis of a biological fluid containing protein-binding substances to be removed, with
- a biological fluid circuit (3) and
- a dialyser (1), wherein in the dialysis fluid circuit (2),
- at least one means (4;6;7;8;9) for solubilizing the protein-binding substances to be removed, and
- at least one dialysis, filtration or diafiltration device (5) are provided,
wherein a dialysis fluid usable in the means contains an adsorber for the substances to be removed from the biological fluid,
**characterized in that** the means (4;6;7;8;9) for solubilizing the protein-binding substances to be removed comprises at least one device (4) for adjusting the pH of the usable dialysis fluid to pH = 8-13.

2. Means according to claim 1 comprising at least one other means (4;6;7;8;9) for solubilizing protein-binding substances to be removed, selected from the following group
- device (4) for adjusting the pH of the usable fluids to pH = 2,5-5,
- a device (6) for adjusting the temperature of the usable fluids,
- a device (7) for adding substituate to dilute or change the salt content of the usable fluids,
- a device (8) for adding dialyzable compounds binding to the substances to be removed,
- a device (9) for irradiating the usable fluids with waves.

3. Means according to claim 1 or 2, **characterized in that** at least one means (4;6;7;8;9) for solubilizing the protein-binding substances to be removed is provided in the biological fluid circuit (3).

4. Means according to one of the preceding claims, **characterized in that** the device (4) for adjusting the pH of the usable fluids comprises a device for adding base and a device for adding acid.

5. Means according to one of the preceding claims, **characterized in that** the device (6) for adjusting the temperature comprises heating or cooling devices.

6. Means according to claim 5, **characterized in that** the heating device (6) comprises a heating apparatus, a micro-wave apparatus or an infrared apparatus, and the cooling device (6) is a cooling unit.

7. Means according to claim 2, **characterized in that** the irradiating device (9) comprises an ultrasonic apparatus.

8. Means according to one of the preceding claims, **characterized in that** at least one other dialysis, filtration or diafiltration device (5) is provided in the biological fluid circuit (3).

9. Means according to one of the preceding claims, **characterized in that** a device (6) for heating the biological fluid to 45°C, and a device (6) for cooling the usable fluids are provided in the biological fluid circuit (3).

10. Means according to one of the preceding claims, **characterized in that,** upstream from the entrance to the dialysis fluid circuit (2) or the biological fluid circuit (3), the heating device (6) is inserted downstream of a device (4) for adjusting the pH or a device (7) for adding substituate.

11. Means according to one of the preceding claims, **characterized in that** the adsorber comprises albumin.

12. Means according to claim 11, **characterized in that** the dialysis fluid contains human serum albumin in a concentration of 1 to 25 g per 100 ml, preferably of 2 to 10 g per 100 ml and particularly preferably of 4 to 6 g per 100 ml.

13. Means for the dialysis of a biological fluid containing protein-binding substances to be removed, according to any one of claims 1-10, wherein the dialysis fluid usable in the means does not contain an adsorber for the substances to be removed from the biological fluid.

## Revendications

1. Dispositif pour la dialyse d'un liquide biologique, qui contient des substances liées à des protéines, à éliminer, avec
- une circulation (3) du liquide biologique, et
- un dialyseur (1),
- - au moins un moyen (4 ; 6 ; 7 ; 8 ; 9) pour la mise en solution des substances liées à des protéines, à éliminer,
- - au moins une installation (5) pour la dialyse, la filtration ou la diafiltration,
étant prévus dans la circulation du liquide de dialyse (2),
un liquide de dialyse utilisable dans le dispositif, contenant un adsorbant pour les substances à éliminer du liquide biologique,
**caractérisé en ce que** le moyen (4 ; 6 ; 7 ; 8 ; 9) pour la mise en solution des substances liées à des protéines, à éliminer, comporte au moins une installation (4) pour l'ajustement du pH du liquide de dialyse utilisable à une valeur de 8-13.

2. Dispositif selon la revendication 1, comportant au moins un autre moyen (4 ; 6 ; 7 ; 8 ; 9) pour la mise en solution de substances liées à des protéines, à éliminer, choisi dans le groupe constitué d'une :
- installation (4) pour l'ajustement du pH des liquides utilisables, à une valeur de 2,5 à 5,
- installation (6) pour l'ajustement de la température des liquides utilisables,
- installation (7) pour l'addition d'un substitut pour la dilution ou la modification de la teneur en sel des liquides utilisables,
- installation (8) pour l'addition de composés dialysables, liant les substances à éliminer,
- installation (9) pour l'irradiation des liquides utilisables avec des ondes.

3. Dispositif selon la revendication 1 ou 2, **caractérisé en ce que** dans la circulation (3) du liquide biologique, est prévu au moins un moyen (4 ; 6 ; 7 ; 8 ; 9) pour la mise en solution des substances liées à des protéines, à éliminer.

4. Dispositif selon l'une quelconque des revendications précédentes, **caractérisé en ce que** l'installation (4) pour l'ajustement du pH des liquides utilisables comporte une installation pour l'addition d'une base et une installation pour l'addition d'un acide.

5. Dispositif selon l'une quelconque des revendications précédentes, **caractérisé en ce que** l'installation (6) pour l'ajustement de la température comporte des installations pour le chauffage ou pour le refroidissement.

6. Dispositif selon la revendication 5, **caractérisé en ce que** l'installation (6) pour le chauffage comporte un appareil de chauffage, un appareil à micro-ondes ou un appareil infrarouge, et **en ce qu'**une machine de refroidissement constitue l'installation (6) pour le refroidissement.

7. Dispositif selon la revendication 2, **caractérisé en ce que** l'installation (9) pour l'irradiation comporte un appareil à ultra-sons.

8. Dispositif selon l'une quelconque des revendications précédentes, **caractérisé en ce que** dans la circulation (3) du liquide biologique, est prévue au moins une autre installation (5) pour la dialyse, la filtration ou la diafiltration.

9. Dispositif selon l'une quelconque des revendications précédentes, **caractérisé en ce que** dans la circulation (3) du liquide biologique, sont prévues une installation (6) pour le chauffage du liquide biologique jusqu'à 45°C et une installation (6) pour le refroidissement des liquides utilisables.

10. Dispositif selon l'une quelconque des revendications précédentes, **caractérisé en ce que** l'installation (6) pour le chauffage avant l'entrée dans la circulation du liquide de dialyse (2) ou dans la circulation (3) du liquide biologique est disposée à la suite d'une installation (4) pour l'ajustement de la valeur du pH ou à la suite d'une installation (7) pour l'addition du substitut.

11. Dispositif selon l'une quelconque des revendications précédentes, **caractérisé en ce que** l'adsorbant comporte de l'albumine.

12. Dispositif selon la revendication 11, **caractérisé en ce que** le liquide de dialyse contient de la sérumalbumine humaine en une concentration allant de 1 à 25 g par 100 ml, de préférence allant de 2 à 10 g par 100 ml, allant en particulier de 4 à 6 g par 100 ml.

13. Dispositif pour la dialyse d'un liquide biologique, qui contient des substances liées à des protéines, à éliminer, selon l'une quelconque des revendications 1 à 10, le liquide de dialyse utilisable dans le dispositif ne contenant pas d'adsorbant pour les substances à éliminer du liquide biologique.
